# EUROPEAN PATENT APPLICATION

(11) **EP 4 046 581 A1**
(43) Date of publication of application: **24.08.2022**
(21) Application number: 22168049.9
(22) Date of filing: 05.03.2020
(51) Int. Cl.: A61B 17/70

(54) **A BOLT APPARATUS FOR VERTEBRAL FIXATION**

(30) Priority: 05.03.2019 US 201962813991 P
(62) Divisional of application: 20711084.2
(71) Applicant: Sota Orthopaedics Ltd., Malahide, Co. Dublin (IE)
(72) Inventor: THORNES, Brian, Sutton (IE); MCDONALD, Ross, Howth (IE)
(74) Representative: FRKelly

(57) **Abstract**

The present invention relates to a bolt apparatus for vertebral fixation. In particular, the apparatus finds utility as a bolt apparatus for fixation of bones of the vertebral column (or spine or backbone), each bone known as a vertebra. In certain embodiments, the bolt apparatus has a body comprising an expandable section and expanding means to displace the expandable section between a contracted position and an expanded position; and a cross-section of at least part of the body is non-circular.

## Description

### Field of the Invention

The present invention relates to a bolt apparatus for vertebral fixation. In particular, the apparatus finds utility as a bolt apparatus for fixation of bones of the vertebral column (or spine or backbone), each bone known as a vertebra.

### Background to the Invention

Each vertebra of the vertebral column comprises an anterior-facing vertebral body and a posterior-facing vertebral arch, which together define the vertebral foramen, a closed aperture enclosing the spinal canal, through which the spinal cord passes. The vertebral arch comprises two pedicles, two laminae, and seven processes. The pedicles connect the vertebral body and the vertebral arch.

Pedicle screw fixation is commonly used in spinal instrumentation surgeries to connect rods to vertebrae in order to correct spine alignment, stabilize vertebrae, and reach an arthrodesis. Indeed, trans-pedicle screw fixation is the gold standard of vertebral fixation, such that pedicle screw fixation is commonplace for securing anchorage in thoraco-lumbar spinal surgery in order to treat a variety of conditions, including degenerative, deformity, tumour and trauma applications.

In pedicle screw fixation, pedicle screws are placed from the posterior aspect of the spine. After surgical exposure, the anatomical landmarks are identified. The posterior cortical crest of bone is removed, for example with a rongeur or burr, to expose the underlying cancellous bone. The entry point is prepared, preferably with an awl. Using a curved blunt probe, a pathway into the pedicle is created. The probe should follow a path of least resistance without violating the pedicle walls. If resistance is felt, the entry point and trajectory should be re-evaluated. At any stage, correct position can be confirmed, for example with fluoroscopy. Other instruments, such as a pedicle feeler or drilling tool (such as PediGuard^{®} by SpineGuard) can also be used to assist positioning and/or trajectory. For increased bone purchase, bone taps can be used to prepare the pedicle canal.

The pedicle screw head has a large U-shaped component to engage with the posterior spinal rods. This pedicle screw head can either be monoaxial, polyaxial or uniplanar, to facilitate the ease of, and appropriate connection with, the posterior metal rods. The pedicle screws are inserted and gain anchorage in the pedicle in the subcortical (cancellous) bone and to a lesser extent in the vertebral body in the cancellous bone.

A pedicle screw relies mostly on a short segment of anchorage between screw and subcortical (cancellous) bone on the inner aspect of the pedicle. Extreme care must be taken to avoid breaching the cortical wall of the pedicle, so as not to enter the spinal canal and injure the spinal cord and/or nerve roots. If the cortical bone is breached, then subsequent anchorage with a pedicle screw is greatly weakened. Anchorage may also be difficult in revision surgery and in patients with osteoporotic or pathological bone. Loss of screw anchorage (pull-out) or screw breakage typically occurs from micro-movement in the flexion-extension plane, with possibly small additional rotational stresses.

To be effective, a pedicle screw must withstand intraoperative loading and physiological forces due to daily postoperative activities. The pedicle screw needs to resist flexion-extension forces and to a lesser extent, lateral flexion forces and rotational torque forces. The cross section of a pedicle is oblong, longer in superior-inferior dimension than medio-lateral dimension, but pedicle screws by their nature are circular in cross section and therefore cannot maximise the anatomical resources of bone for fixation.

It is an object of the present invention to provide an improved device for vertebral fixation, which provides a means for reducing the occurrence of screw pull-out and screw breakage, and which can resist flexion-extension forces, lateral flexion forces and/or rotational torque forces.

### Summary of the Invention

According to the present invention there is provided a bolt apparatus for fixation of spinal bones, the bolt apparatus having a body, the body comprising:
(a) an expandable section having respective ends, the expandable section being operable between a contracted position and an expanded position;
(b) expanding means in operable association with the expandable section, to displace the expandable section between the contracted position and the expanded position by applying force to the respective ends of the expandable section, such that each of the respective ends of the expandable section are advanced toward the opposing respective end;
wherein a cross-section of at least part of the body is non-circular.

Optionally, the cross-section of at least part of the body is ovate. Further optionally, the cross-section of at least part of the body is elliptical. Further optionally, the cross-section of at least part of the body is oviform. Alternatively, the cross-section of at least part of the body is obround.

Optionally, the body has a first end and a second end. Further optionally, the body has first and second opposing ends.

Optionally, the cross-section of the second end of the body is non-circular.

Optionally, the (a) expandable section and the (b) expanding means are located at the first end of the body. Further optionally, the (a) expandable section and the (b) expanding means are located at the first end of the body, and the cross-section of the second end of the body is non-circular. Still further optionally, the body has first and second opposing ends, wherein the (a) expandable section and the (b) expanding means are located at the first end of the body, and the cross-section of the second end of the body is non-circular.

Optionally, the cross-section of the second end of the body is ovate.

Further optionally, the (a) expandable section and the (b) expanding means are located at the first end of the body, and the cross-section of the second end of the body is ovate. Still further optionally, the body has first and second opposing ends, wherein the (a) expandable section and the (b) expanding means are located at the first end of the body, and the cross-section of the second end of the body is ovate.

Optionally, the bolt apparatus further comprises at least one fin. Further optionally, the bolt apparatus further comprises at least two fins. Still further optionally, the bolt apparatus further comprises at least three fins. Still further optionally, the bolt apparatus further comprises at least four fins.

Optionally, the at least one fin projects from the body.

Optionally, the at least one fin is located at the second end of the body.

Optionally, the bolt apparatus further comprises at least two fins, wherein each fin projects from the body and is located at the second end of the body. Further optionally, the bolt apparatus further comprises at least a pair of fins, wherein each fin projects from the body and is located at the second end of the body. Optionally, each fin in each pair of fins is located diametrically opposed to the other fin in the pair of fins.

Optionally, the at least one fin is tapered. Further optionally, the at least one fin is tapered with respect to the longitudinal axis of the body. Still further optionally, the at least one fin is tapered toward the first end of the body with respect to the longitudinal axis of the body.

Optionally, the expanding means displaces the expandable section between the contracted position and the expanded position by simultaneously applying force to the respective ends of the expandable section, such that each of the respective ends of the expandable section are advanced toward the opposing respective end. As used herein, the term "advanced" is intended to mean the positive displacement of an object between a first position and a second position, wherein the first and second positions are different, spaced-apart positions. It is understood that, in the present invention, the first position of each respective end is the position when the expandable section is in the fully expanded position, and the second position of each respective end is the position when the expandable section is in the fully contracted position. Each respective end is displaced by the application of a force to each of the respective ends of the expandable section. Each respective end can optionally be simultaneously displaced by the simultaneous application of a force to each of the respective ends of the expandable section.

Optionally, the expanding means is in operable association with the expandable section, to displace the expandable section between the contracted position and the expanded position by applying force to the respective ends of the expandable section, such that each of the respective ends of the expandable section are independently advanced toward the opposing respective end. As used herein, the term "independently advanced" is intended to mean the positive and independent displacement of an object between a first position and a second position, wherein the first and second positions are different, spaced-apart positions. Each respective end is displaced by the application of a force to each of the respective ends of the expandable section. Each respective end can be displaced by the independent application of a force to each of the respective ends of the expandable section.

Further optionally, the expanding means is in operable association with the expandable section, to displace the expandable section between the contracted position and the expanded position by simultaneously applying force to the respective ends of the expandable section, such that each of the respective ends of the expandable section are independently advanced toward the opposing respective end. Each respective end is displaced by the application of a force to each of the respective ends of the expandable section. Each respective end can be optionally simultaneously displaced by the independent and simultaneous application of a force to each of the respective ends of the expandable section.

Preferably, the expanding means comprises a connecting means and at least two bodies mountable to the connecting means.

Preferably, the connecting means is adapted to allow reciprocal movement of the at least two bodies relative to the connecting means.

Preferably, the connecting means is a shaft.

Preferably, the at least two bodies are threadably mountable to the connecting means, for example the shaft. Further preferably, first and second bodies are threadably mountable to the connecting means, and arranged for displacement in response to rotation of the connecting means to apply mechanical pressure to the respective ends of the expandable section. The connecting means can be arranged to simultaneously and independently apply mechanical pressure to the respective ends of the expandable section. The at least two bodies are displaced in response to rotation of the connecting means relative to one, both or each of the at least two bodies.

Preferably, the connecting means comprises first and second threaded portions, wherein the threads of the first portion are of reverse orientation to the threads of the second portion.

Preferably, the first body is mountable to the first threaded portion of the connecting means and the second body is mountable to the second threaded portion of the connecting means.

Optionally, the first and second threaded portions are located adjacent one end, for example a distal end, of the connecting means. Alternatively, the first and second threaded portions are located adjacent respective opposing ends of the connecting means.

Preferably, the threaded portions of the connecting means are helically threaded portions.

Optionally, a proximal end of the connecting means is dimensioned and arranged, for example by way of a transverse cross-section, so as to inhibit the coaxial rotation of the shaft relative to a set screw, once assembled.

Optionally, the proximal end of the connecting means is dimensioned and arranged, so as to provide means for delivering torque to the connecting means. Preferably, the proximal end of the connecting means is dimensioned and arranged to receive a torque delivery device such as a screwdriver, or similar device. Alternatively, the proximal end of the connecting means is dimensioned and arranged to allow rotation thereof by a hex key, or similar device.

Optionally, one, both, or each of the at least two bodies is engagable with at least one of the respective ends of the expandable section. Further optionally, one, both, or each of the at least two bodies is irreversibly engagable with at least one of the respective ends of the expandable section.

Optionally, one, both, or each of the at least two bodies is integral with the body. Further optionally, one, both, or each of the at least two bodies is integral with at least one of the respective ends of the expandable section.

Optionally, one, both, or each of the at least two bodies is integral with the body and comprises a screw threaded portion located on the inner surface of the body. Further optionally, one, both, or each of the at least two bodies is integral with the body and comprises a screw threaded portion located on the inner surface of at least one of the respective ends of the expandable section.

Preferably, the expanding means comprises a connecting means comprising first and second threaded portions, wherein the threads of the first portion are of reverse orientation to the threads of the second portion; and a first body mountable to the first threaded portion of the connecting means; and a second screw threaded portion located on the inner surface of the body and mountable to the second threaded portion of the connecting means.

Preferably, the expandable section is reversibly expandable. More preferably, the section is reversibly expandable under mechanical pressure.

Preferably, the expandable section is collapsible along its longitudinal axis. Further preferably, the expandable section is radially inwardly collapsible.

Preferably, the expandable section comprises at least two expandable members that extend from the longitudinal axis of the apparatus under mechanical pressure. More preferably, the expandable members extend radially from the longitudinal axis of the apparatus under mechanical pressure.

Preferably, each of the expandable members comprises a deformable arm.

Preferably, at least one point of folding is provided along each deformable arm.

Preferably, the or each point of folding comprises a point of weakness, a hinge mechanism, or any such mechanism that will facilitate the folding of the deformable arm at a desired location.

Optionally, the cross-section of the expandable section is circular. Further optionally, the expandable section is located at the first end of the body and the cross-section of the expandable section is circular.

Preferably, the bolt apparatus is formed of a material that is suitable for sterilisation, so as to be provided in a sterile packaged state for use.

Preferably, the material is autoclavable.

Preferably the material is surgical stainless steel, but it will be seen that any material that is suitable for sterilisation and can impart the required mechanical strength may be used. For example, the material can be titanium alloy, but may also be pure titanium, or other medically approved metal or material.

For the purposes of the present specification, a user is a person who will undertake the operation of the device during routine use. Usually, this will be a medical professional, where routine use includes fixation of a bone of a patient. When in use, the invention is oriented so as to have a proximal end and a distal end relative to said user.

A patient is defined as a person on whom the device will be used during routine operation.

### Brief Description of the Drawings

Embodiments of the invention will now be described, by way of example, with reference to the accompanying drawings, in which:

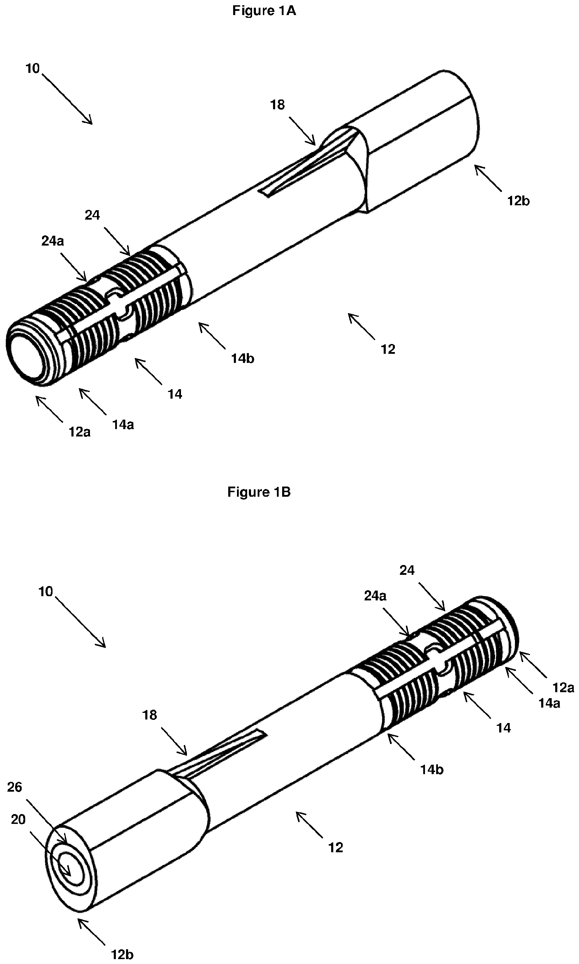

**Figure 1A** is a perspective view of a distal end of a bolt apparatus according to a first embodiment of the present invention;
**Figure 1B** is a perspective view of a proximal end of the bolt apparatus of Figure 1 ;

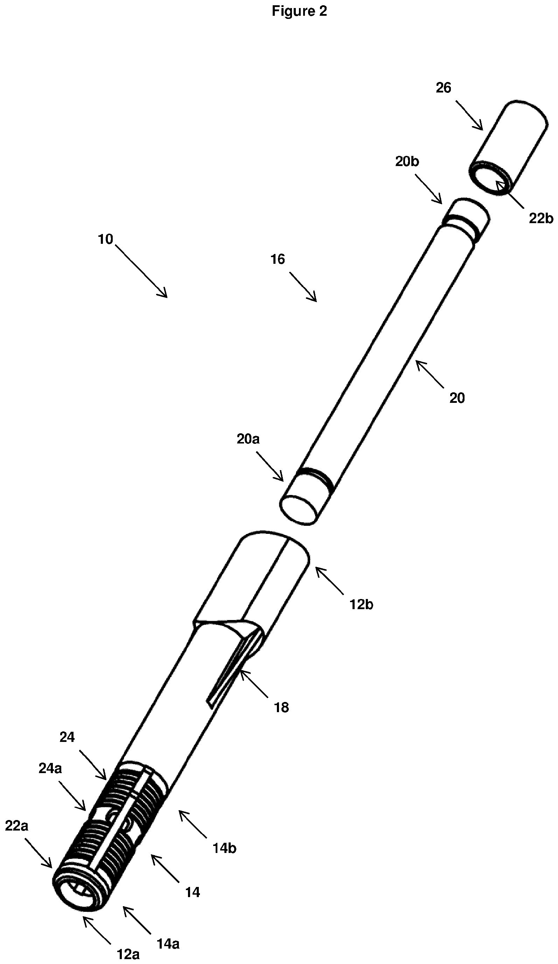

**Figure 2** is an exploded perspective view of the bolt apparatus of Figure 1;

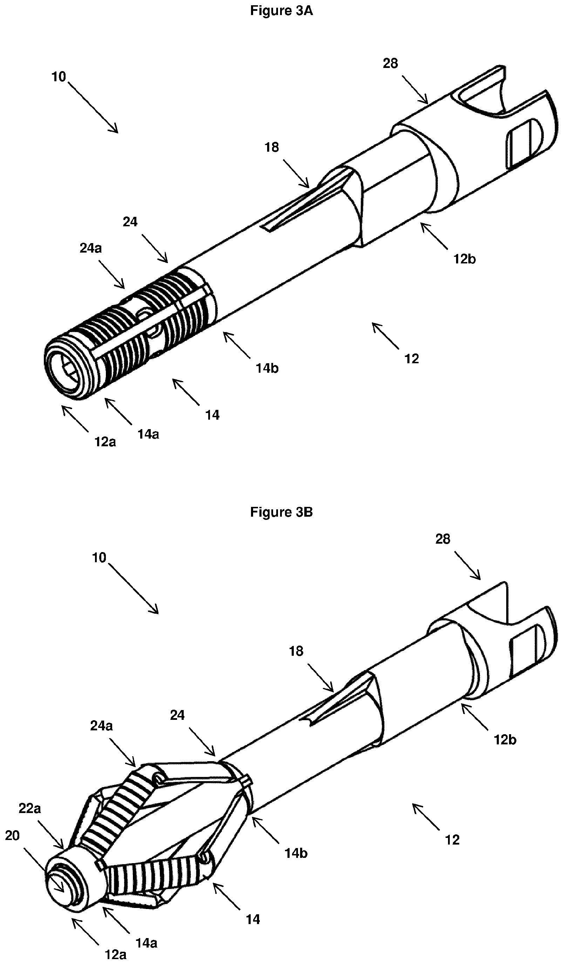

**Figure 3A** is a perspective view of a bolt apparatus according to a second embodiment of the present invention with the expandable section in a contracted position; and
**Figure 3B** is a perspective view of a bolt apparatus according to a second embodiment of the present invention with the expandable section in an expanded position.

In the drawings, similar reference numerals will be used to indicate like parts.

### Detailed Description of the Invention

Referring now to the drawings, there is shown a bolt apparatus 10 according to a preferred embodiment of the invention. The bolt apparatus 10 has a body 12.

The body 12 comprises an expandable section 14 operable between a contracted position and an expanded position; and expanding means 16 in operable association with the expandable section 14. The expanding means 16 can displace the expandable section 14 between the contracted position and the expanded position by applying force to the respective ends of the expandable section 14, such that each of the respective ends of the expandable section 14 are advanced toward the opposing respective end.

A cross-section of at least part of the body 12 is non-circular.

In this embodiment, the cross-section of at least part of the body 12 is ovate, but the cross-section of at least part of the body 12 can be elliptical or oviform. In another embodiment, the cross-section of at least part of the body 12 is obround.

In this embodiment, the body 12 has first 12a and second 12b opposing ends. The expandable section 14 and the expanding means 16 are located at a first end 12a of the body 12. The cross-section of the second end 12b of the body 12 is non-circular, in this embodiment ovate.

The bolt apparatus 10 further comprises at least one fin 18. Each of the at least one fins 18 projects from the body. Each of the at least one fins 18 is located at the second end 12b of the body 12. In this embodiment, the bolt apparatus further comprises a pair of fins 18a, 18b. Each fin 18a, 18b projects from the body 12 and is located at the second end 12b of the body 12. Although not essential, each fin 18a in the pair is located diametrically opposed to the other fin 18b in the pair. Each fin 18 is tapered, preferably tapered with respect to the longitudinal axis of the body 12. In this preferred embodiment, the at least one fin 18 is tapered toward the first end 12a of the body 12.

The expanding means 16 displaces the expandable section 14 between the contracted position and the expanded position by simultaneously applying force to the respective ends 14a, 14b of the expandable section 14, such that each respective end 14a of the expandable section 14 is advanced toward the opposing respective end 14b. Each opposing respective end 14a, 14b can be simultaneously displaced by the independent and simultaneous application of a force to each of the respective ends 14a, 14b of the expandable section 14.

Preferably, the expanding means 16 comprises a connecting means 20 and at least two bodies 22a, 22b mountable to the connecting means 20. The connecting means 20 can be a shaft 20, which comprises an elongate member, and which is generally cylindrical in shape. The connecting means 20 is adapted to allow reciprocal movement of the at least two bodies 22a, 22b relative to the connecting means 20. The at least two bodies 22, 22b can be threadably mountable to the connecting means 20 and arranged for displacement in response to rotation of the connecting means 20 to simultaneously and independently apply mechanical pressure to the respective ends 14a, 14b of the expandable section 14.

In the preferred embodiment, the connecting means 20 is a shaft 20, which comprises first 20a and second 20b threaded portions, wherein the threads of the first portion 20a are of reverse orientation to the threads of the second portion 20b. Preferably, the threaded portions 20a, 20b of the connecting means 20 are helically threaded portions.

Preferably, the first body 22a is mountable to the first threaded portion 20a of the connecting means 20, and the second body 22b is mountable to the second threaded portion 20a of the connecting means 20.

In this embodiment, the first 20a and second 20b threaded portions are located adjacent one end, for example a distal end, of the connecting means 20.

In this preferred embodiment, the body 12 comprises a tube, which is generally cylindrical in shape and is open at each end. The inner surface of the body 12 is generally circular in transverse cross-section. The internal diameter of the body 12 is generally of similar length to the external diameter of the shaft 20, whereby the shaft 20 can be located longitudinally and rotated coaxially within the body 12.

In this preferred embodiment, one of the first body 22a is integral to the body 12 and comprises a helical screw thread provided along a limited length of the inner surface of the body 12.

The helical screw thread provided along a limited length of the inner surface of the body 12 can engage with one of the screw threaded portions 20a, 20b of the shaft 20. Specifically, a first helical screw thread provided along a limited length of the inner surface of the body 12 engages with the first screw threaded portion 20a of the shaft 20.

The proximal end of the connecting means 20 can be dimensioned and arranged to receive a torque delivery device such as a screwdriver, or similar device, so as to provide means for delivering torque to the connecting means 20. In an alternative embodiment, the proximal end of the connecting means 20, for example the shaft 20 is dimensioned and arranged to allow rotation thereof by a hex key, or similar device. However, it will be appreciated that any shape of screw drive to deliver the required torque may be used.

One, both, or each of the at least two bodies 22a, 22b can be engaged with at least one of the respective ends 14a, 14b of the expandable section 14. In such an embodiment, the expandable section 14 can be reversibly expandable, for example under mechanical pressure. Additionally, the expandable section 14 can be collapsible along its longitudinal axis, for example the expandable section 14 can be radially inwardly collapsible.

The expandable section 14 comprises expandable members 24, for example deformable arms 24, which extend from the longitudinal axis of the apparatus 10 under mechanical pressure. The expandable member 24, for example a deformable arm 24, extends radially from the longitudinal axis of the apparatus under mechanical pressure. In a preferred embodiment, at least one point of folding 24a is provided along each deformable arm 24. Each point of folding 24a acts as a point of weakness, a hinge mechanism, or any such mechanism to facilitate the folding of the deformable arm 24 at a desired location. A point of weakness 24a can be provided at each of the respective ends of each deformable arm 24, where the deformable arm 24 is attached to the respective ends of the expandable section 14; and adjacent the centre point of the length of each deformable arm 24.

The cross-section of the expandable section 14 is circular. The expandable section 14 is located at the first end 12a of the body 12 in a preferred embodiment and the cross-section of the expandable section 14 is circular.

The assembly comprising the shaft 20 and the mountable bodies 22a, 22b is located coaxially within the body 12.

Once assembled, each of the mountable bodies 22a, 22b is located on a respective screw threaded portion 20a, 20b of the shaft 20. Each of the mountable bodies 22a, 22b is in tandem but opposite orientation relative to the other mountable body, and is located at each respective end of the expandable section 14. Each of the mountable bodies 22a, 22b can be attached to each respective end 14a, 14b of the expandable section 14 by an adherent means, such as an adhesive.

In the preferred embodiment, to assemble the bolt apparatus 10, the second mountable body 22b is located onto the second screw threaded portion 20b of the shaft 20. The shaft 20 is then inserted coaxially into the lumen of the body 12, and the first screw threaded portion 20a of the shaft 20 is engaged with a helical screw thread on the inner surface of the body 12, such that the second mountable body 22b is located coaxially within, and irreversibly engages with, the lumen of the body adjacent the distal end.

Rotation of the shaft 20 within the body 12 causes each of the mountable bodies 22a, 22b to respectively advance along the screw threaded portions 20a, 20b, respectively, of the shaft 20. This applies mechanical pressure to the respective ends 14a, 14b of the expandable section 14, wherein the respective ends 14a, 14b are bought sequentially into closer proximity relative to one another, causing the deformable arms 24 of the expandable section 14 to deform at each of the points of weakness 24a, and to expand radially from the longitudinal axis of the bolt assembly 10.

The second body 22b can be a set screw 26. The set screw 26 can comprise a helical screw thread provided along a limited length of the inner surface of the set screw 26. The helical screw thread provided along a limited length of the inner surface of the set screw 26 can be threadably mountable to the connecting means 20, for example, to the second threaded portion 20b of the connecting means 20. The set screw 26 can be irreversibly secured, for example welded to the body 12, for example the second or posterior end 12b of the body 12.

Rotation of the shaft 20 in the opposite direction can ultimately cause the deformable arms 24 to retract toward the longitudinal axis of the bolt assembly 10, thereby facilitating the removal of the device, if required.

The bolt apparatus 10 is preferably formed of a material that is suitable for sterilisation, such as an autoclavable material, so as to be provided in a sterile packaged state for use. The material can be surgical stainless steel, but it will be seen that any material that is suitable for sterilisation and can impart the required mechanical strength may be used.

In a method for vertebral fixation; the bolt apparatus 10, once assembled and with the expandable section 14 in a contracted position, is placed from the posterior aspect of the spine. After surgical exposure, the anatomical landmarks are identified. The posterior cortical crest of bone is removed, for example with a rongeur or burr, to expose the underlying cancellous bone. The entry point is prepared, preferably with an awl. Using a curved blunt probe, a pathway into the pedicle is created. The probe should follow a path of least resistance without violating the pedicle walls. If resistance is felt, the entry point and trajectory should be re-evaluated. At any stage, correct position can be confirmed, for example with fluoroscopy. Other instruments, such as a pedicle feeler or drilling tool (such as PediGuard^{®} by SpineGuard) can also be used to assist positioning and/or trajectory.

The pathway in cancellous bone may be enlarged by use of successive reamers of increasing outer diameter, ensuring that the cortical bone of the pedicle or vertebral body is not breached. A starter cavity within the cancellous bone of the vertebral body may be prepared with an instrument such as a curette or a bone compactor, later into which the deformable arms 24 of the expandable section 14 of the body 12 of the bolt apparatus 10 will radially expand.

When the bone pathway has been prepared, the bolt apparatus 10, once assembled and with the expandable section 14 in a contracted position, is inserted from the posterior aspect of the vertebra, through the pedicle and into the vertebral body. The position of the fins 18 should be noted, keeping the fins 18 broadly within the superior-inferior (sagittal) plane. Position may be checked with an image intensifier, noting that the location of the expandable section 14 is appropriately placed within the vertebral body and not in the pedicle.

The bolt apparatus 10 may be advanced gradually by light hammering on the base (posterior end) of the bolt apparatus 10. This may help advance the fins 18 into the cancellous bone of the pedicle.

The deformable arms 24 of the expandable section 14 of the body 12 of the bolt apparatus 10 are radially expanded using, for example, a screwdriver inserted into, for example, a hex socket in the proximal end of the connecting means 20, for example the shaft 20, with counter rotation being resisted by an instrument, such as a spanner, placed at the posterior end 12b of the bolt apparatus 10. Expansion is continued until a mechanical stop is felt, or until the torque limiter of the screwdriver trips out, or from operator choice from visual feedback on the image intensifier.

Whilst the expandable section 14 is being expanded, the posterior end 12b of the bolt apparatus 10 will be pulled anteriorly, deeper into the cancellous bone. This will advance further the fins 18 into the cancellous bone of the pedicle in a controlled manner with ergonomic feedback to the user. The fins 18 provide additional fixation strength and rotational stability to the bolt apparatus 10. The fins 18 have the advantage of gaining additional fixation within the ovate pedicle that would otherwise not be utilised by a circular cross-sectioned screw.

Should undue resistance be felt via the screwdriver, or torque limit of the screwdriver tripped out, the user may reassess position of the bolt apparatus 10 in relation to the cortical bone of the pedicle and vertebral body. If necessary, the expansion may be reversed by counter-clockwise rotation of the screwdriver and the bolt apparatus 10, with the expandable section 14 in the contracted position, removed.

The bolt apparatus 10 has a posterior end 12b, which can be monoaxial, polyaxial or uniplanar, to facilitate the ease of, and appropriate connection with, posterior metal rods. The posterior end 12b of the bolt apparatus 10 can comprise a large U-shaped component 28 to engage with posterior spinal rods.

### Embodiments

1. A bolt apparatus for fixation of spinal bones, the bolt apparatus having a body,
   the body comprising:
      (a) an expandable section having respective ends, the expandable section being operable between a contracted position and an expanded position;
      (b) expanding means in operable association with the expandable section, to displace the expandable section between the contracted position and the expanded position by applying force to the respective ends of the expandable section, such that each of the respective ends of the expandable section are advanced toward the opposing respective end;
   wherein a cross-section of at least part of the body is non-circular.
2. A bolt apparatus according to Embodiment 1, wherein the cross-section of at least part of the body is ovate.
3. A bolt apparatus according to Embodiment 1, wherein the body has a first end and a second end, and the cross-section of the second end of the body is non-circular.
4. A bolt apparatus according to Embodiment 3, wherein the (a) expandable section and the (b) expanding means are located at the first end of the body.
5. A bolt apparatus according to Embodiment 3 or 4, wherein the bolt apparatus further comprises at least one fin, wherein the at least one fin projects from the body.
6. A bolt apparatus according to Embodiment 5, wherein the at least one fin is located at the second end of the body.
7. A bolt apparatus according to Embodiment 5 or 6, wherein the bolt apparatus comprises at least two fins.
8. A bolt apparatus according to any one of Embodiments 5-7, wherein the at least one fin is tapered toward the first end of the body with respect to the longitudinal axis of the body.
9. A bolt apparatus according to any one of Embodiments 1-8, wherein the expanding means displaces the expandable section between the contracted position and the expanded position by simultaneously applying force to the respective ends of the expandable section, such that each of the respective ends of the expandable section are advanced toward the opposing respective end.
10. A bolt apparatus according to any one of Embodiments 1-9, wherein the expanding means is in operable association with the expandable section, to displace the expandable section between the contracted position and the expanded position by applying force to the respective ends of the expandable section, such that each of the respective ends of the expandable section are independently advanced toward the opposing respective end.
11. A bolt apparatus according to any one of Embodiments 1-10, wherein the expanding means comprises a connecting means and at least two bodies mountable to the connecting means.
12. A bolt apparatus according to Embodiment 11, wherein the connecting means is a shaft.
13. A bolt apparatus according to Embodiment 11 or 12, wherein the at least two bodies are threadably mountable to the connecting means.
14. A bolt apparatus according to any one of Embodiments 11-13, wherein the connecting means comprises first and second threaded portions, wherein the threads of the first portion are of reverse orientation to the threads of the second portion.
15. A bolt apparatus according to any one of Embodiments 1-14, wherein the expandable section comprises at least two expandable members that extend from the longitudinal axis of the apparatus under mechanical pressure.

## Claims

1. A bolt apparatus for fixation of spinal bones, the bolt apparatus having a body, the body comprising:
(a) an expandable section having respective ends, the expandable section being operable between a contracted position and an expanded position;
(b) expanding means in operable association with the expandable section, to displace the expandable section between the contracted position and the expanded position by applying force to the respective ends of the expandable section, such that each of the respective ends of the expandable section are advanced toward the opposing respective end;
wherein the bolt apparatus further comprises at least one fin.

2. A bolt apparatus according to claim 1, wherein the at least one fin projects from the body.

3. A bolt apparatus according to claim 1 or 2, wherein the body has first and second opposing ends, and the at least one fin is located at the second end of the body.

4. A bolt apparatus according to any one of claims 1-3, wherein the bolt apparatus comprises at least a pair of fins, wherein each fin projects from the body and is located at the second end of the body.

5. A bolt apparatus according to any one of claims 1-4, wherein each fin in each pair of fins is located diametrically opposed to the other fin in the pair of fins.

6. A bolt apparatus according to any one of claims 1-5, wherein the at least one fin is tapered.

7. A bolt apparatus according to any one of claims 1-6, wherein the at least one fin is tapered toward the first end of the body with respect to the longitudinal axis of the body.

8. A bolt apparatus according to any one of claims 3-7, wherein the (a) expandable section and the (b) expanding means are located at the first end of the body.

9. A bolt apparatus according to any one of claims 1-8, wherein the expanding means displaces the expandable section between the contracted position and the expanded position by simultaneously applying force to the respective ends of the expandable section, such that each of the respective ends of the expandable section are advanced toward the opposing respective end.

10. A bolt apparatus according to any one of claims 1-9, wherein the expanding means comprises a connecting means and at least two bodies mountable to the connecting means.

11. A bolt apparatus according to claim 10, wherein the connecting means is adapted to allow reciprocal movement of the at least two bodies relative to the connecting means.

12. A bolt apparatus according to claim 10 or 11, wherein the connecting means comprises first and second threaded portions, wherein the threads of the first portion are of reverse orientation to the threads of the second portion.

13. A bolt apparatus according to any one of claims 10-12, wherein the first body is mountable to the first threaded portion of the connecting means and the second body is mountable to the second threaded portion of the connecting means.

14. A bolt apparatus according to any one of claims 1-13, wherein, the expandable section is reversibly expandable.

15. A bolt apparatus according to any one of claims 1-14, wherein a cross-section of at least part of the body is selected from non-circular, ovate, elliptical, oviform, and obround.
